# EUROPEAN PATENT APPLICATION

(11) **EP 2 230 235 A1**
(43) Date of publication of application: **22.09.2010**
(21) Application number: 09003841.5
(22) Date of filing: 17.03.2009
(51) Int. Cl.: C07D 321/10, A61K 31/357, A61P 35/00, A61P 31/00

(54) **Botryosphaerones, novel depsidones and their use as medicaments**

(71) Applicant: Leibniz-Institut für Naturstoff-Forschung und Infektionsbiologie e.V. -Hans-Knöll-Institut- (HKI), 07745 Jena (DE)
(72) Inventor: Abdou, Randa, 07743 Jena (DE); Dahse, Hans Martin, 07749 Jena (DE); Hertweck, Christian, 04105 Leipzig (DE); Scherlach, Kristin, 04105 Leipzig (DE); Sattler, Isabel, 67663 Kaiserslautern (DE)
(74) Representative: Forstmeyer, Dietmar

(57) **Abstract**

The present invention describes novel compounds (depsidones) of formulas (I) and (1a) as well as their use for the treatment of bacterial infections, fungal diseases (mycosis) or cancer.

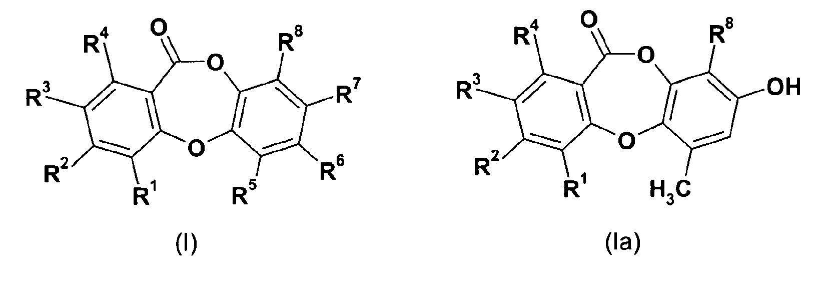

## Description

An endophytic fungus (*Botryosphaeria rhodina*) was isolated from the stems of the medicinal plant *Bidens pilosa* (fam. *Asteraceae*). The ethyl acetate extract of the fungal isolate exhibits significant antifungal activity as well as potent cytotoxic and antiproliferative effects against several cancer cell lines. Activity-guided fractionation resulted in the isolation of compounds having cytotoxic and antifungal activities.

Of the 300,000 plant species that exist on the earth, each individual plant is host to one or more endophytes, thus providing a rich reservoir of microorganisms.¹⁻³ Various endophytic fungi interact in mutualism with their host plant taking advantage of nutrients provided by the host and in turn producing bioactive substances to enhance the growth and competitiveness of the host in nature.^{4, 5} In many cases an improved resistance of the host plant to adversity is observed due to the production of bioactive secondary metabolites by its endophyte.⁵ This benefit is based on a fine-tuned balance between the demands of the invading endophyte and the plant response. If the interaction becomes unbalanced disease symptoms appear or the fungus is excluded by induced host defence reactions.⁶ Furthermore, endophytes have been recognized as a prolific source of a wide array of new pharmacologically active secondary metabolites that might prove suitable for specific medicinal or agrochemical applications.⁶ There is also an urgent need for new antibiotics, chemotherapeutic agents and agrochemicals caused by the development of resistance in infectious microorganisms to existing drugs.¹ *Bidens pilosa* is a herbaceous plant widely distributed in Africa, America, China, and Japan that is used in traditional medicines for treatment of inflammation and various diseases, including hepatitis and diabetes. The boiling water extract of the aerial parts of *Bidens pilosa* in Japan has anti-inflammatory and anti-allergic properties.⁷ Furthermore, the ethanolic crude extract from the roots of *Bidens pilosa* contains polyacetylenes and flavonoids that exert *in vitro* antimalarial activity against *Plasmodium falciparum.*⁸ More recently, a study was carried out to examine the possibility of using *Bidens pilosa* for weed and plant fungus control assuming that the wide distribution of the plant might be due to its antifungal activity against phytopathogens.^{3, 9} All studies performed so far focused on the phenolic constituents of the plant extract, and yet endophytes of *B. pilosa* and their potentially active metabolites have been neglected.

The present invention relates to novel compounds (depsidones), the production of these compounds and to their use as pharmaceuticals, especially for the treatment of cancer, bacterial infections, Cutaneous and systemic fungal infections such as Aspergillosis, Fusariosis and otomycosis affecting immunocompromised patients (AIDS patients and patients receiving chemotherapy or organ transplants).¹⁰⁻¹³ These compounds exhibit significant antifungal activity as well as potent cytotoxic, cytostatic and antiproliferative effects against several cancer cell lines.

The present invention provides compounds of formula (I): 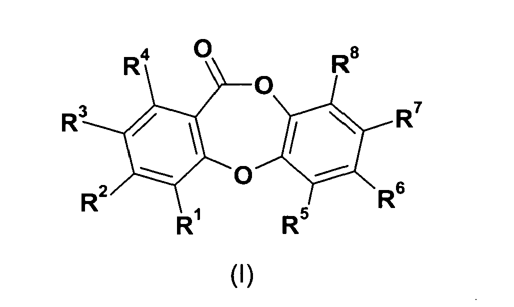 wherein
R¹ is a group of formula CHO or CH₂OH;
R² is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R³ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁴ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁵ is hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁶ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁷ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, and
R⁸ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
or wherein two of the groups R², R³, R⁴, R⁵, R⁶ R⁷ and R⁸ together are part of a cycloalkyl, a heterocycloalkyl, an alkylcycloalkyl, a heteroalkylcycloalkyl, an aryl, a heteroaryl, an aralkyl or a heteroaralkyl group;
with the proviso that at least one of R⁵ or R⁸ is a hydrogen atom when R¹ is CHO,
or a pharmacologically acceptable salt, solvate, hydrate or a pharmacologically acceptable formulation thereof.

The present invention further provides the use of compounds of formula (Ia) 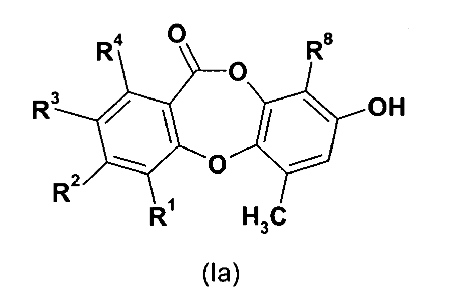 wherein
R¹ is a group of formula CHO or CH₂OH;
R² is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R³ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁴ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁸ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
or wherein two of the groups R², R³, R⁴ and R⁸ together are part of a cycloalkyl, a heterocycloalkyl, an alkylcycloalkyl, a heteroalkylcycloalkyl, an aryl, a heteroaryl, an aralkyl or a heteroaralkyl group,
or a pharmacologically acceptable salt, solvate, hydrate or a pharmacologically acceptable formulation thereof as pharmaceuticals, especially for the treatment of bacterial infections, fungal diseases (e.g. mycosis, aspergillosis, fusariosis, otomycosis) or cancer.

The expression alkyl refers to a saturated, straight-chain or branched hydrocarbon group that contains from 1 to 20 carbon atoms, preferably from 1 to 12 carbon atoms, especially from 1 to 6 carbon atoms, for example a methyl, ethyl, propyl, iso-propyl, n-butyl, iso-butyl, tert-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 carbon atoms, for example an ethenyl, allyl, acetylenyl, propargyl, isoprenyl or hex-2-enyl group. Preferably, alkenyl groups have one or two (especially one) double bond(s) and alkynyl groups have one or two (especially one) triple bond(s).

Furthermore, the terms alkyl, alkenyl and alkynyl refer to groups in which one or more hydrogen atoms have been replaced by a halogen atom (preferably F or Cl) such as, for example, a 2,2,2-trichloroethyl or a trifluoromethyl group.

The expression heteroalkyl refers to an alkyl, alkenyl (e.g. heteroalkenyl) or alkynyl group in which one or more (preferably 1, 2 or 3) carbon atoms have been replaced by an oxygen, nitrogen, phosphorus, boron, selenium, silicon or sulphur atom (preferably oxygen, sulphur or nitrogen). Examples for heteroalkyl groups are alkyloxy, alkyloxyalkyl, alkenyloxyalkyl, alkenyloxy, alkyloxyalkenyl, alkylamino, alkylaminoalkyl, dialkylamino, dialkylaminoalkyl, alkylthio or alkylthioalkyl groups. The expression heteroalkyl furthermore refers to a carboxylic acid or to a group derived from a carboxylic acid such as, for example, acyl, acyloxy, acyloxyalkyl, acylalkyl, alkoxycarbonyl, alkyloxycarbonylalkyl, carboxyalkylamide, alkylcarbonylamino, alkylcarbonylaminoalkyl, alkylaminocarbonyl, alkylaminocarbonylalkyl, or alkoxycarbonyloxy.

Examples of heteroalkyl groups are groups of formulae R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}, R^{a}-N(R^{b})-CS-S-Y^{a}-, R^{a}-S-CS-O-Y^{a}-, R^{a}-O-CS-S-Y^{a}-(preferred are R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}- and R^{a}-N(R^{b})-CO-Y^{a}-,),
R^{a} being a hydrogen atom, a C₁-C₆alkyl, a C₂-C₆alkenyl or a C₂-C₆alkynyl group; R^{b} being a hydrogen atom, a C₁-C₆alkyl, a C₂-C₆alkenyl or a C₂-C₆alkynyl group; R^{c} being a hydrogen atom, a C₁-C₆alkyl, a C₂-C₆alkenyl or a C₂-C₆alkynyl group; R^{d} being a hydrogen atom, a C₁-C₆alkyl, a C₂-C₆alkenyl or a C₂-C₆alkynyl group and Y^{a} being a bond, a C₁-C₆alkylene, a C₂-C₆alkenylene or a C₂-C₆alkynylene group (preferrably, R^{a}, R^{b}, R^{c} and R^{d} are independently H or C₁-C₆ alkyl and Y^{a} is a bond or C₂-C₆alkylene),
each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups.

The expression cycloalkyl refers to a saturated or partially unsaturated (for example cycloalkenyl), cyclic group that contains one or more rings (preferably 1 or 2), containing from 3 to 50 carbon atoms, preferably 3 to 14, further preferably from 3 to 10 (especially 3, 4, 5, 6 or 7) carbon atoms. The expression cycloalkyl refers furthermore to groups in which one or more hydrogen atoms have been replaced by fluorine, chlorine, bromine or iodine atoms or by OH, =O, SH, =S, NH₂, =NH or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulphur atom (preferably oxygen, sulphur or nitrogen). A heterocycloalkyl group has preferably 1 or 2 ring(s) containing from 3 to 10 (especially 3, 4, 5, 6 or 7) ring atoms. The expression heterocycloalkyl refers furthermore to groups in which one or more hydrogen atoms have been replaced by fluorine, chlorine, bromine or iodine atoms or by OH, =O, SH, =S, NH₂, =NH or NO₂ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also lactams, lactones, cyclic imides and cyclic anhydrides.

The expression alkylcycloalkyl refers to groups containing both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings containing from 3 to 10 (especially 3, 4, 5, 6 or 7) carbon atoms, and one or two alkyl, alkenyl or alkynyl groups having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (especially 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenyl-heterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl or Ar refers to an aromatic group that has one or more rings containing from 6 to 50 carbon atoms, preferably 6 to 14, further preferably from 6 to 10 (especially 6) carbon atoms. The expression aryl (or Ar) refers furthermore to groups in which one or more hydrogen atoms have been replaced by fluorine, chlorine, bromine or iodine atoms or by OH, SH, NH₂ or NO₂ groups. Examples are a phenyl, naphthyl, biphenyl, 2-fluorophenyl, anilinyl, 3-nitrophenyl or 4-hydroxyphenyl group.

The expression heteroaryl refers to an aromatic group that has one or more rings containing from 5 to 50 ring atoms, preferably 5 to 14, further preferably from 5 to 10 (especially 5 or 6) ring atoms, and contains one or more (preferably 1, 2, 3 or 4) oxygen, nitrogen, phosphorus or sulphur ring atoms (preferably O, S or N). The expression heteroaryl refers furthermore to groups in which one or more hydrogen atoms have been replaced by fluorine, chlorine, bromine or iodine atoms or by OH, SH, NH₂ or NO₂ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1 H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced by oxygen, sulphur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups in which one or more hydrogen atoms of such groups have been replaced by fluorine, chlorine, bromine or iodine atoms or by OH, =O, SH, =S, NH₂, =NH or NO₂ groups.

Furthermore, all alkyl, alkenyl, alkynyl, heteroalkyl and especially all cycloalkyl, heterocycloalkyl, alkylcycloalkyl, heteroalkylcycloalkyl, aryl, heteroaryl, aralkyl and heteroaralkyl groups as defined herein may be optionally substituted.

The expression "optionally substituted" refers to groups in which one or more hydrogen atoms have been replaced by fluorine, chlorine, bromine or iodine atoms or by OH, =O, SH, =S, NH₂, =NH or NO₂ groups. This expression refers furthermore to groups that are substituted by one or more (e.g. 1, 2 or 3) unsubstituted C₁-C₆alkyl, C₂-C₆alkenyl, C₂-C₆alkynyl, C₁-C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.

Owing to their substitution, compounds of formula (I) or (Ia) may contain one or more centres of chirality. The present invention therefore includes both all pure enantiomers and all pure diastereoisomers and also mixtures thereof in any mixing ratio. The present invention moreover also includes all cis/trans-isomers of the compounds of the general formula (I) or (Ia) and also mixtures thereof. The present invention moreover includes all tautomeric forms of the compounds of formula (I) or (Ia).

Preferred are compounds of formula (I) or (Ia) wherein R¹ is an aldehyde (CHO) group.

Further preferred are compounds of formula (I) or (Ia) wherein R² is a hydroxy or a methoxy group, especially a hydroxy group.

Moreover preferred are compounds of formula (I) or (Ia) wherein R³ is a hydrogen atom or a halogen atom (e.g. F or Cl), more preferably, H or Cl, especially a hydrogen atom.

Further preferred are compounds of formula (I) or (Ia) wherein R⁴ is a C₁-C₄ alkyl group or a C₂-C₄ alkenyl group, especially a methyl group.

Further preferred are compounds of formula (I) wherein R⁵ is a C₁-C₄ alkyl group, especially a methyl group.

Further preferred are compounds of formula (I) wherein R⁶ is a hydrogen atom, a halogen atom (e.g. F or Cl), CHO or COOH, especially hydrogen.

Further preferred are compounds of formula (I) wherein R⁷ is a hydroxy or a methoxy group, especially a hydroxy group.

Further preferred are compounds of formula (I) or (Ia) wherein R⁸ is a hydrogen atom or a C₁-C₄ alkyl group, especially hydrogen or a methyl group.

It is to be noted that the present invention also encompasses all possible combinations of all preferred embodiments.

Especially preferred, the compound of formula (I) or (Ia) has one of the following structures:

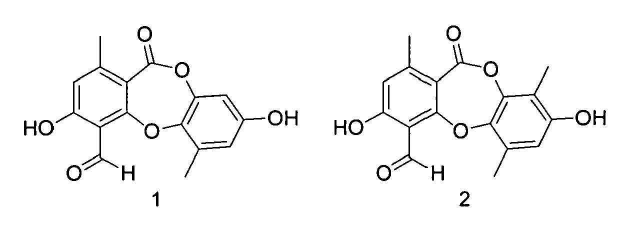

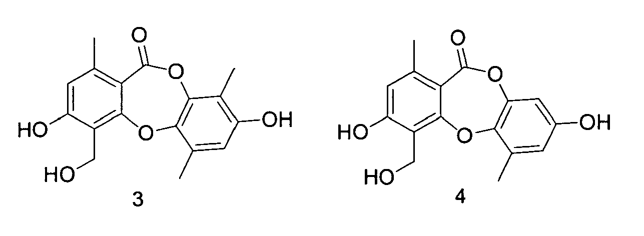

The therapeutic use of compounds of formula (I) or (Ia), their pharmacologically acceptable salts or solvates and hydrates and also formulations and pharmaceutical compositions also lie within the scope of the present invention, especially, the use of compounds of formula (I) or (Ia), their pharmacologically acceptable salts or solvates and hydrates and also formulations and pharmaceutical compositions for the treatment of bacterial infections, fungal diseases (e.g. mycosis aspergillosis, fusariosis, otomycosis) or cancer as well as their use for the preparation of medicaments for the treatment of bacterial infections, fungal diseases (e.g. mycosis aspergillosis, fusariosis, otomycosis) or cancer. Packaged pharmaceutical preparations are also provided, comprising such a pharmaceutical composition in a container and instructions for using the composition to treat a patient suffering from one of these diseases.

The pharmaceutically compositions according to the present invention comprise at least one compound of formula (I) or (Ia) as active ingredient and, optionally, carrier substances and/or adjuvants. Further, these pharmaceutically compositions may comprise other antimicrobial, antifungal, antiproliverative, cytostatic and/or cytotoxic ingredients.

Examples of pharmacologically acceptable salts of the compounds of formula (I) or (Ia) are salts of physiologically acceptable mineral acids, such as hydrochloric acid, sulphuric acid and phosphoric acid, or salts of organic acids, such as methanesulphonic acid, p-toluenesulphonic acid, lactic acid, acetic acid, trifluoroacetic acid, citric acid, succinic acid, fumaric acid, maleic acid and salicylic acid. Further examples of pharmacologically acceptable salts of the compounds of formula (I) or (Ia) are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. Compounds of formula (I) or (Ia) may be solvated, especially hydrated. The hydration may take place, for example, during the preparation process or as a consequence of the hygroscopic nature of the initially anhydrous compounds of formula (I) or (Ia). When the compounds of formula (I) or (Ia) comprise asymmetric C-atoms, they may be present either in the form of achiral compounds, diastereoisomeric mixtures, mixtures of enantiomers or in the form of optically pure compounds.

The pro-drugs to which the present invention also relates consist of a compound of formula (I) or (Ia) and at least one pharmacologically acceptable protecting group which will be removed under physiological conditions, such as, for example, an alkoxy-, aralkyloxy-, acyl- or acyloxy group, such as, for example, an ethoxy, benzyloxy, acetyl or acetyloxy group.

The present invention relates also to the use of those active ingredients in the preparation of medicaments. In general, compounds of formula (I) or (Ia) are administered either individually, or in combination with any other desired therapeutic agent, using the known and acceptable methods. Such therapeutically useful agents may be administered, for example, by one of the following routes: orally, for example in the form of dragées, coated tablets, pills, semi-solid substances, soft or hard capsules, solutions, emulsions or suspensions; parenterally, for example in the form of an injectable solution; rectally in the form of suppositories; by inhalation, for example in the form of a powder formulation or a spray; transdermally or intranasally. For the preparation of such tablets, pills, semi-solid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

For the prevention and/or treatment of bacterial infections, fungal diseases (e.g. mycosis, aspergillosis, fusariosis, otomycosis) or cancer, the dose of the biologically active compound according to the invention may vary within wide limits and may be adjusted to individual requirements. Generally, a dose of from 10 mg to 4000 mg per day is suitable, a preferred dose being from 50 to 3000 mg per day. In suitable cases, the dose may also be below or above the stated values. The daily dose may be administered as a single dose or in a plurality of doses. A typical individual dose contains approximately 50 mg, 100 mg, 250 mg, 500 mg, 1 g or 2 g of the active ingredient.

Further, the compounds described in the present invention may be used as pesticides.

Samples of *B. pilosa* were collected near Cairo, Egypt. After surface sterilization of the fresh aerial plant parts an endophytic fungal strain was isolated. The strain was identified as *Botryosphaeria rhodina* (also known as *Lasiodiplodia theobromae*) on the basis of the ITS sequence^{14, 15}. To monitor the production of secondary metabolites of the isolate it was cultivated in four different culture media both as stationary and as shaken cultures. The resulting extracts were subjected to antimicrobial activity studies and cytotoxicity assays. It has been found that the extract from a medium 25 stationary culture exhibited the highest antimicrobial and antiproliferative activities. The ethyl acetate extract of the endophyte exhibited significant antifungal activity against various test strains in agar diffusion assays (*Sporobolomyces salmonicolor, Saccharomyces cerevisiae, Candida albicans, Penicillium notatum, Penicillium avellanea,* and *Aspergillus terreus*). To identify and characterize the active compounds large scale fermentation (40 L) of the endophyte was carried out under the optimized culture condition. The crude extract was liberated from lipophilic components and subjected to flash chromatographic separation on silica gel, followed by open column chromatography with Sephadex LH-20. Activity-guided fractionation was performed by testing the resulting fractions against *A. terreus,* which proved to be the most sensitive test strain towards the crude extract.

Active fractions contained compounds **1** and **2,** which are chemically related as based on retention time and UV spectra, and belong to a complex of four aromatic metabolites **(1-4).** These four compounds were isolated by open column chromatography and repeated preparative RP-HPLC using an acetonitrile / water gradient, yielding **1** (8 mg), **2** (5 mg), **3** (2 mg) and **4** (3 mg).

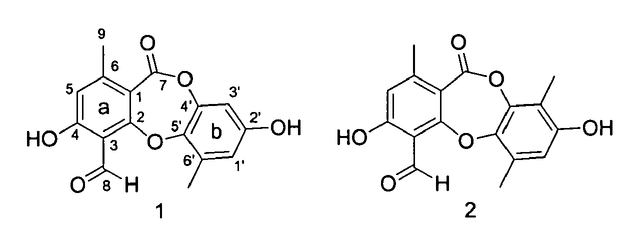

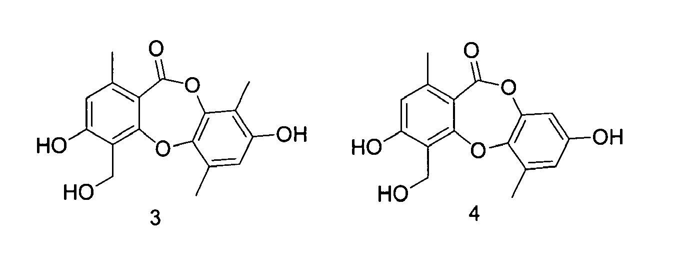

Compound **1** has a molecular formula of C₁₆H₁₂O₆ as indicated by HRESIMS and ¹³C NMR data (Table 1). The ¹H NMR spectrum exhibited signals corresponding to two aromatic methyl groups (δ 2.26; 2.41), three aromatic protons (δ 6.47; 6.54; 6.59), one hydroxyl proton (δ 9.79) and one aldehyde proton (δ 10.57). The ¹³C-NMR spectrum revealed the presence of 16 carbons of which 2 were methyl groups (δ 16.7; 21.4), one aldehyde carbonyl ( 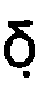 191.9) and one ester carbonyl group ( 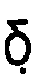 164.5). From HSQC and HMBC data the presence of two aromatic phenyl groups was concluded. HMBC data indicated that the first ring contained a methyl group (δ 2.41), which was correlated with a methine carbon at 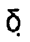 116.9 (C-5) and an ester carbonyl carbon (C-7) at δ 164.5. Furthermore, the aldehyde moiety, which was also confirmed by IR (1683 cm⁻¹), also correlated to the same carbon C-5 and to an OH-bonded carbon (C-4, δ 163.8). The strong downfield shift of the OH-bearing carbon (C-4) could be referred to H-bond chelation with the adjacent aldehyde group, thus indicating the architecture of the first phenyl group (a).

The second phenyl group is substituted with two *meta* coupled protons at 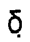 6.54 (H-3') and 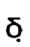 6.47 (H-1') as well as a methyl group at δ 16.7 which was correlated with a methine carbon (C-1') at δ 114.2 and two quaternary carbons (C-6') at 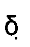 131.2 and (C-5') at 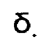 141.2 on the basis of HMBC correlations. The presence of another oxygen-bound carbon was deduced from the correlations observed for the proton at δ 6.54 (H-3') with two quaternary carbons, C-5' at δ 141.2 and C-4' at 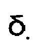 144.0 through which the second phenyl group was completely deduced. Contrary to the downfield shift observed for the oxygen bonded carbons C-2, C-5' and C-4' an upfield shift was observed for C-1 ( 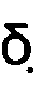 111.5), thus suggesting its connection to a carbonyl carbon. Consequently, both phenyl groups are connected by a seven-membered ring containing an ether linkage and an ester bridge, revealing compound **1** to be a new depsidone, for which the name botryosphaerone A was proposed.

**Table 1. NMR spectroscopic data (DMSO-d6) for botryosphaerones A (1) and B (2). ^{a}HMBC correlations, optimized for 6 Hz, are from protons stated to the indicated carbon.**

| Botryosphaerone A **(1)** | | | | Botryosphaerone B **(2)** | | |
|---|---|---|---|---|---|---|
| position | δ_{c,} mult. | δ_{H} (*J* in Hz) | HMBC^{a} | δ_{c,} mult. | δ_{H} (*J* in Hz) | HMBC^{a} |
| 1 | 111.5, qC | | | 114.0, qC | | |
| 2 | 161.8, qC | | | 163.4, qC | | |
| 3 | 112.3, qC | | | 112.1, qC | | |
| 4 | 163.8, qC | | | 165.4, qC | | |
| 5 | 116.9, CH | 6.59, s | 2,3,4,9 | 118.1, CH | 6.73, s | 1, 3, 9 |
| 6 | 152.0, qC | | | 155.6, qC | | |
| 7 | 164.5, qC | | | 166.4, qC | | |
| 8 | 191.9; CH | 10.57, s | 4,5 | 194.6, CH | 10.71, s | 4,5 |
| 9 | 21.4, CH₃ | 2.41, s | 2.3,5,6,7 | 22.3, CH₃ | 2.48, | 1,5,6 |
| 1' | 114.2, CH | 6.47, d (3.0) | 2',3',4',5',7' | 114.3, CH | 6.46, s | 3',5',7' |
| 2' | 155.1, qC | | | 155.0, qC | | |
| 3' | 105.3, CH | 6.54, d (3.0) | 1',2',4',5' | 116.1, qC | | |
| 4' | 144.0, qC | | | 145.3, qC | | |
| 5' | 141.2, qC | | | 144.0, qC | | |
| 6' | 131.2, qC | | | 128.3, qC | | |
| 7' | 16.7, CH₃ | 2.26, s | 1',3',4',5',6' | 17.1, CH₃ | 2.30, s | 1',5',6' |
| 8' | | | | 9.2, CH₃ | 2.19, s | 2',3',4',6' |
| OH | | 9.79 | | | | |

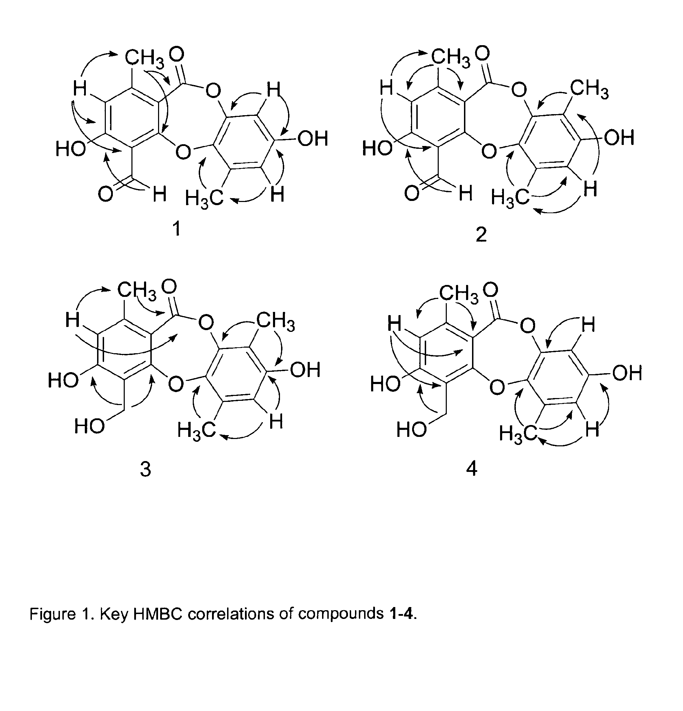

Compound **2** was obtained as a second product with significant antifungal activity. From HRESIMS and ¹³C NMR data the molecular formula C₁₇H₁₄O₆ was deduced, thus suggesting that **2** is a homologue of **1** with an additional methyl group. The ¹³C NMR signal at δ 194.6 and the IR absorption band at 1683 cm⁻¹ confirmed the presence of the aldehyde function. Its proton was correlated with an OH-bearing quaternary carbon at δ 165.4 and a methine carbon (C-5, 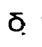 118.1) on the basis of HMBC correlations. This proton (H-5) is in turn correlated with its neighboring methyl carbon at δ 22.3, as well as a quaternary carbon connected to the ester carbonyl group (C-1) at 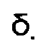 114.0 on the basis of HMBC (Table 1, Figure 1). The second aromatic ring bears two methyl groups, a hydroxyl group and a single proton, as deduced from the ¹³C NMR, HSQC and HMBC correlations. One of the methyl groups (C-8') is correlated with the OH-bearing carbon (C-2', 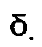 155.0) and the quaternary carbon bound to an oxygen atom (C-4', 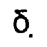 145.3). The protons of the second methyl group on the other hand are correlated with the methine aromatic carbon (C-1', δ 114.3), the quaternary carbon bound to the same methyl group (C-6', δ 128.3) and a carbon bound to an oxygen atom (C-5', 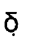 +144.0). This information fully established the substitution pattern of **2.**

Compound **3** (botryosphaerone C) appeared as the second major compound of the extract. It exhibited antibacterial activity against *Bacillus subtilis* in agar diffusion assays (400 µg mL⁻¹). Its molecular formula was determined as C₁₇H₁₆O₆ on the basis of HRESIMS and ¹³C NMR data. The spectral data of compound **3** (Table 2) are similar to those of **1** indicating that both have the same basic skeleton. However, the ¹³C NMR spectrum lacks an aldehyde signal, but shows a methylene signal (δ 54.8, DEPT). The chemical shift suggested that this methylene is located in the periphery of a phenyl and is connected to a hydroxyl group. This was further supported by the HMBC correlations (Figure 1) between the methylenec protons and the carbons C-2 ( 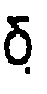 162.5), C-4 bearing an OH group at 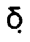 163.0 and the quaternary carbon (C-3) at 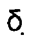 117.0. Compound **3** also differs from **1** in the additional methyl group at C-3', which is also present in **2**.

**Table 2. NMR spectroscopic data (DMSO-d6) for botryosphaerones C (3) and D (4). ^{a}HMBC correlations, optimized for 6 Hz, are from protons stated to the indicated carbon.**

| Botryosphaerone C **(3)** | | | | Botryosphaerone D **(4)** | | |
|---|---|---|---|---|---|---|
| position | δc, mult. | δH (J in Hz) | HMBCa | δc, mult. | δH (J in Hz) | HMBCa |
| 1 | 113.6, qC | | | 113.7, qC | | |
| 2 | 162.5, qC | | | 162.5, qC | | |
| 3 | 117.0, qC | | | 117.0, qC | | |
| 4 | 163.0, qC | | | 162.8, qC | | |
| 5 | 116.2, CH | 6.59, s | 1,2,3,7,9 | 116.2, CH | 6.60, s | 1,3,9 |
| 6 | 145.9, qC | | | 146.1, qC | | |
| 7 | 166.0, qC | | | 165.7, qC | | |
| 8 | 54.8, CH2 | 4.95, s | 2,3,4 | 54.7, CH2 | 4.94, s | 3,4 |
| 9 | 21.4, CH3 | 2.38, s | 2,5,6,7 | 21.4, CH3 | 2.37, s | 1, 5, 6 |
| 1' | 114.1, CH | 6.43, s | 2',3',4',7',8' | 115.2, CH | 6.44, | 2', 4', 7' |
| 2' | 153.9, qC | | | 155.9, qC | | |
| 3' | 115.3, qC | | | 105.9, CH | 6.44, s | 2',4',5' |
| 4' | 144.7, qC | | | 143.7, qC | | |
| 5' | 144.1, qC | | | 145.8, qC | | |
| 6' | 128.6, qC | | | 132.8, qC | | |
| 7' | 16.9, CH3 | 2.39, s | 1',5',6' | 17.2, CH3 | 2.43, s | 1',3',4',5',6' |
| 8' | 9.2, CH3 | 2,12, s | 2',3',4',6' | | | |

Compound **4** (botryosphaerone D,), a minor product from the fraction of compound **3,** has a molecular formula of C₁₆H₁₄O₆ as indicated by HRESIMS and ¹³C NMR. The ¹H NMR and ¹³C NMR spectral data (Table 2) showed close similarity to those of compound **3** suggesting that both compounds have the same basic framework. In contrast to compound **3** the ¹H NMR shows only two methyl proton signals (δ 2.37, δ 2.43) but three aromatic proton signals of which one appeared at 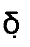 6.60 and two at δ 6.44. On the basis of HMBC correlations (Table 2) it was confirmed that the C-8' methyl group of **3** is lacking as in the b-ring of **1.**

The compounds described in the present invention exert a variety of biological actions including antibiotic, antimycobacterial, antiviral, anti-inflammatory, analgesic, antipyretic, anti-proliferative and cytotoxic effects.¹⁶ Further, these compounds act as photoprotectors limiting the deleterious effects of UV through their antioxidant activity.^{17, 18} Furthermore, they modulate microbial populations living within or attacking plants.¹⁹

The antimicrobial efficacy of compounds **1-2** has been evaluated against test strains *A. terreus* and *B. subtilis.* Compounds **1** and **2** are active against both microorganisms. Compounds **3** and **4** show antibacterial activity (MIC 400 µg mL⁻¹ for **3).** The minimum inhibitory concentration of compound **1** against *A. terreus* was found to be 7.81 µg mL⁻¹, while the reference nystatin has a MIC value of 12.50 µg mL⁻¹. Compound **2** also showed antifungal activity against *A*. *terreus* (MIC 15.63 µg mL⁻¹). These results are particularly interesting since they show that these drugs are of therapeutic value in the treatment of aspergillosis and fusariosis as well as other systemic and cutaneous fungal infections affecting immunocompromised patients worldwide.¹⁰⁻¹³

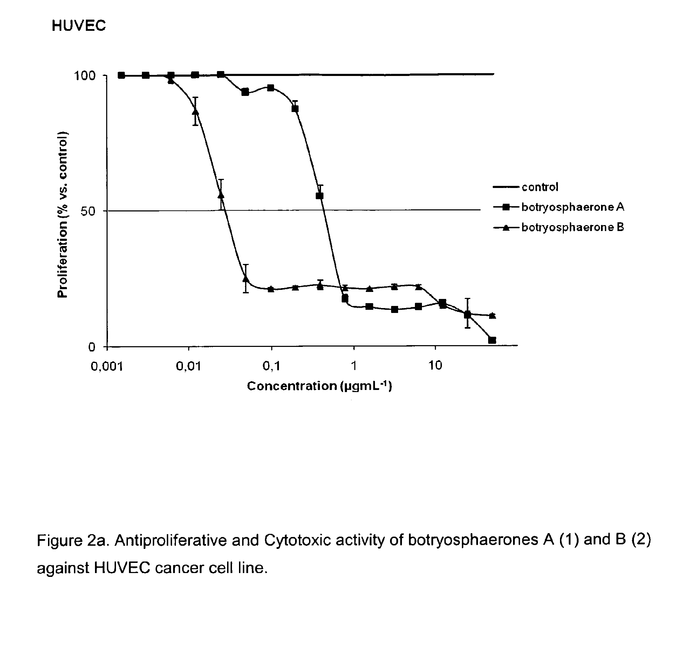

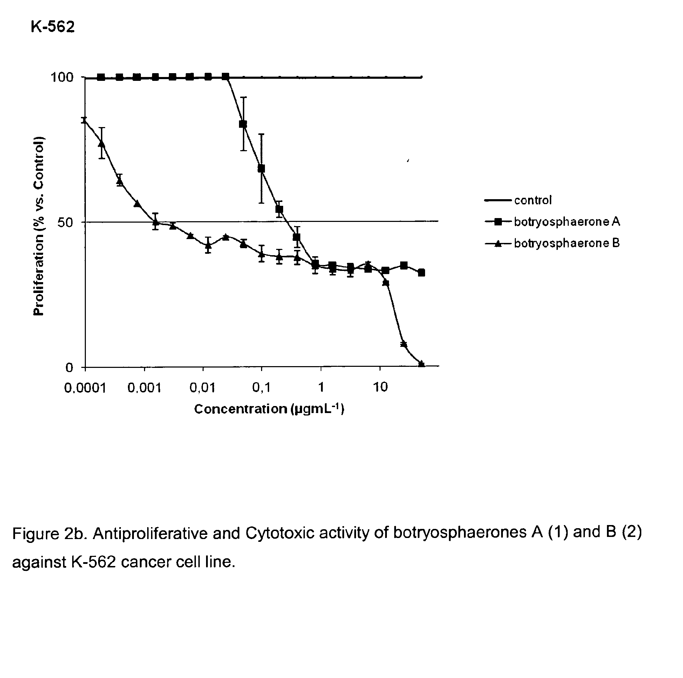

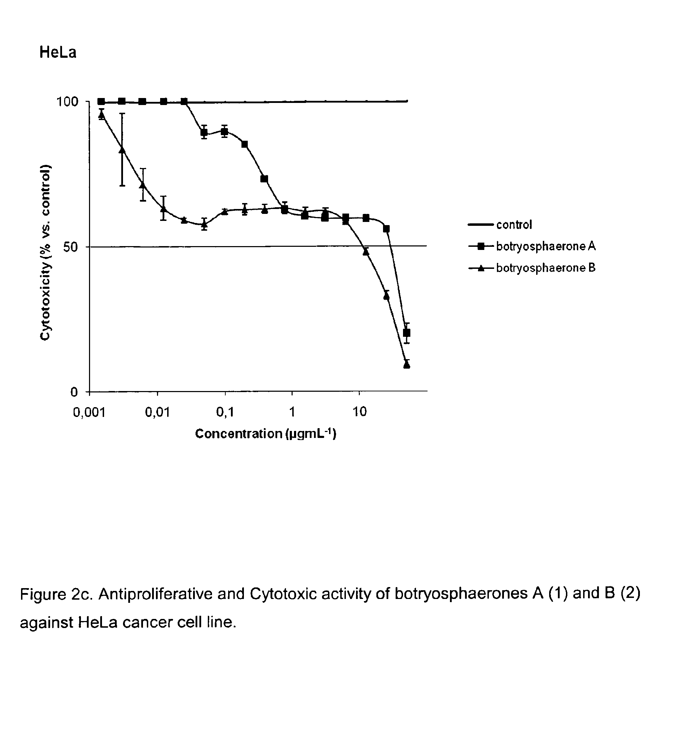

To evaluate the cytotoxicity and antiproliferative effects of **1** and **2** they were subjected to cytotoxicity assays against HeLa, K-562 and HUVEC cell lines. Compound **1** and **2** exhibited potent antiproliferative activity against K-562 and HUVEC (Table 3, Figure 1). In both assays, the two compounds showed significant antiproliferative activity over a wide concentration range with **2** covering the wider range (Figure 2). As for the cytotoxic assay against HeLa cancer cell line CC₅₀ values of 96.97 µM and 36.41 µM were obtained for compound **1** and **2** respectively.

**Table 3. Antiproliferative (Gl₅₀) and cytotoxic (CC₅₀) activities of botryosphaerones A (1) and B (2).**

| Compound | Antiproliferative activity GI₅₀ (HUVEC) | Gl₅₀ (K-562) | Cytotoxicity CC₅₀ (HeLa) |
|---|---|---|---|
| 1 | 1.67 µM | 0.84 µM | 96.97 µM |
| 2 | 0.07 µM | 0.003 µM | 36.41 µM |

The observed antifungal activity of **1** and **2** is intriguing as endophytes may be producing bioactive substances that may be involved in a host-endophyte relationship and can have the capacity to control plant pathogens.⁹ To test whether the endophyte metabolites have the potential to protecting the host plant from fungal infections, they were also tested against the phytopathogen *Fusarium oxysporum.* Indeed, **1** and **2** were active with a MIC of 57.5 µg mL⁻¹ and 75 µg mL⁻¹ observed for **1** and **2** respectively.

It seems that the plant-microbe system presented here belongs to those where the endophyte is aiding the plant as well as itself in its survival and fitness. By producing two antifungal and two antibacterial compounds the endophytic fungus thus provides broad spectrum antimicrobial activity to protect itself from competing invaders and / or the plant from phytopathogens. This is in agreement with previous reports of suppressed pathogenic attack, removed contaminants and promoted plant growth and yield by endophytic fungi.¹⁹

Fusarium oxysporum is also known to be an opportunistic human pathogen reported of causing fusariosis in cancer and AIDS patients and is considered the most frequent fungal agent after Aspergillus attacking patients undergoing bone marrow transplantations.¹²

**General Experimental Procedures:** NMR spectra were recorded on a Bruker DPX-300 and a Bruker DRX-500 at 300 and 500 MHz for ¹H, and 125 MHz for ¹³C NMR, respectively; chemical shifts are given in 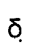 values (ppm). IR spectra were recorded on a Bruker FT-IR (IFS 55) spectrometer. UV spectra were recorded on a Cary 1 Bio UV-visible spectrophotometer (Variant). HPLC-MS measurements were recorded on a Finnigan Surveyor PDA detector/Finnigan TSQ quantum instrument. HRESIMS were recorded on a Finnigan TSQ Quantum Ultra AM sector field mass spectrometer with a compatible ion source. Open column chromatography was performed on silica gel 60 (Merck, 0.04-0.063 mm, 230-400 mesh ASTM) and Sephadex LH-20 (Pharmacia). TLC: silica gel plates (silica gel 60F₂₅₄ on aluminum foil or glass, Merck), spots were visualized by spraying with anise aldehyde/sulfuric acid followed by heating. Analytical HPLC was conducted on a Shimadzu HPLC system using a Nucleosil 100-5 C₁₈ column (5 µm, 125 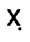 4.6 mm) with MeCN/0.1 % TFA-H₂O as eluent (flow rate 1 mL min⁻¹, 15/85 to 100 % MeCN in 30 min) and UV detection at 254 nm. Preparative HPLC was performed on a Shimadzu HPLC system with a UV detector. All solvents used were spectral grade or distilled prior to use.

**Strain isolation and Taxonomic Classification:** The fungus was isolated from a surface sterilized stem part of the herb *Bidens pilosa* fam. *Asteraceae,* which has been collected near Cairo, Egypt. It was identified on the basis of ITS sequence as *Botryosphaeria rhodina* at the Centraalbureau voor Schimmelcultures in the Netherlands.

**Endophyte Fermentation, Extraction and Isolation:** To monitor the production of secondary metabolites the fungus was cultured in four different media, a malt extract, caseine-flesh peptone, cornsteep and dextrose-yeast medium both as a shaken and stationary cultures after which the antifungal activity of each extract was examined. Both the chemical and biological analyses showed that the antimicrobial activity of the fungal extract and the production of secondary metabolites were highest using a stationary culture (21 days) at 25 °C of M25, a cornsteep medium composed of sucrose (20 g L⁻¹), soy bean starch (10 g L⁻¹) and cornsteep (10 g L⁻¹). The fungus was developed on potato dextrose agar (PDA) at 23° C for 14 days and the mycelium of each plate was cut into 12 pieces each of which was used as an inoculum in a 1 L Erlenmeyer flask, containing 250 mL of M25. Incubation was carried out for 21 days (23° C) under static conditions. The extract from a 40 L culture was prepared by homogenizing the culture filtrate and the mycelium and then macerating for 24 h in EtOAc, which was then removed by decantation. After evaporating to dryness and defatting with n-hexane 10 g of crude extract was obtained. The major products of the extract were compounds **1, 2** and **3.** Activity-guided isolation started by subjecting the extract to fractionation on a silica gel column using (hexane: EtOAc / 1:1) as eluent which resulted in 9 main fractions. The fractions exhibiting antimicrobial activity were successively purified on silica gel using (CHCl₃: MeOH / 9:1); Sephadex LH-20 (MeOH) and finally RP-18 Silica on preparative HPLC starting gradient elution with 25 % acetonitrile in H₂O and ending with 100 % acetonitrile after 45 minutes, to give compounds **1** (8 mg), **2** (5 mg), **3** (2 mg) and **4** (3 mg). The plant growth promoting auxin indole acetic acid was identified by comparison of its chromatographic, UV, IR and MS data with an authentic sample.

**Botryosphaerone A (1):** yellowish-white amorphous powder; UV (MeOH) λ ₘₐₓ (logε) 203 (3.63), 220 (4.32), 326 (sh) (0.43) nm; IR (KBr disk) vₘₐₓ 3651, 2886, 2332, 1683, 1456, 1197, 1147, 1061, 850, 727, 669 cm⁻¹; HRESIMS m/z 299.0547 [M-H]⁻ calcd m/z 299.0550 [M-H]⁻ for C₁₆H₁₁O₆; NMR data see table 1.

**Botryosphaerone B (2):** white amorphous powder; UV (MeOH) λ ₘₐₓ (logε) 207 (3.87), 227 (3.22), 328 (sh) (0.43); IR (KBr disk) vₘₐₓ 3442, 2888, 2346, 1683, 1446, 1207, 1143, 847, 726, 669 cm⁻¹; HRESIMS m/z 313.0722 [M-H]⁻ calcd m/z 313.0707 [M-H]⁻ for C₁₇H₁₃O₆; NMR data see table 1.

**Botryosphaerone C (3):** white amorphous powder; UV (MeOH) λ ₘₐₓ (logε) 209 (3.72), 271 (1.16); IR (KBr disk) vₘₐₓ 3335, 2340, 1670, 1450, 1199, 1146, 849, 726, 666 cm⁻¹; HRESIMS m/z 315.0858 [M-H]⁻ calcd m/z 315.0863 [M-H]⁻ for C₁₇H₁₅O₆; NMR data see table 2.

**Botryosphaerone D (4):** yellowish white amorphous powder; UV (MeOH) λ ₘₐₓ (loge) 217 (4.043); 269 (2.73); IR (KBr disk) vₘₐₓ 3306, 2889, 2340, 1678, 1460, 1200, 1147, 850, 727, 667 cm⁻¹; HRESIMS m/z 301.0705 [M-H]⁻ calcd m/z 301.0707 [M-H]⁻ for C₁₆H₁₃O₆; NMR data see table 2.

### Antimicrobial assay:

Antifungal activities were studied qualitatively by agar diffusion tests according to the literature (Stuttgart, D. A. V., European Pharmacopoeia, 3rd ed.1997, 13, 118; Afonin, S.; Glaser, R. W.; Berditchevskaia, M.; Wadhwani, P.; Guhrs, K. H.; Mollmann, U.; Perner, A.; Ulrich, A. S. Chembiochem. 2003, 4, (11), 1151-1163; Standards, N. C. f. C. L., Reference Method for Broth Dilution Susceptibility Testing of Filamentous Fungi; Approved Standard. M38-A, Wayne, PA, USA, NCCls 2002**,** *22*) and quantitatively by determination of minimal inhibitory concentration (MIC) according to the NCCLS guidelines using the broth microdilution method. Fifty microliters of the test compound solution in methanol were serially diluted by factor two with the culture medium (RPMI 1640 with L-glutamine, MOPS and without sodium bicarbonate, LONZA Verviers SPRL, Belgium). Then, the wells were inoculated with 50 µL of the test organism to give a final concentration of 6x10³ CFU mL⁻¹. After incubation of the microtiter plates at 37°C (*Aspergillus terreus*) for 24 h, the MIC-values were read with a Nepheloscan Ascent 1.4 automatic plate reader (Lab systems, Vantaa, Finnland) as the lowest dilution of compound allowing no visible growth. The MIC for *Fusarium oxysporum* and *Bacillus subtilis* was determined by the agar diffusion method. Fifty microliters of each of the 12 serial twofold dilutions were filled in agar (malt extract agar from Roth, Karlsruhe, Germany; seeded with 0.5 mL of a pretested mycelial solution) holes of 9 mm in diameter. After incubation for 24 h the MIC was read as the lowest concentration giving an inhibition zone.

**Antiproliferative and Cytotoxic Assays:** *Cells and culture conditions.* Cells of HUVEC (ATCC CRL-1730), K-562 (DSM ACC 10) and HeLa (DSM ACC 57) were cultured in DMEM (CAMBREX 12-614F), RPMI 1640 (CAMBREX 12-167F) and RPMI 1640 (CAMBREX 12-167F) respectively. All cells were grown in the appropriate cell culture medium supplemented with 10 mL L⁻¹ ultraglutamine 1 (Cambrex 17-605E/U1), 500 µL L⁻¹ gentamicin sulfate (CAMBREX 17-518Z), and 10 % heat inactivated fetal bovine serum (PAA A15-144) at 37 °C in high density polyethylene flasks (NUNC 156340).

*Antiproliferative assay.* The test substances were dissolved in DMSO before being diluted in DMEM. The adherent cells were harvested at the logarithmic growth phase after soft trypsinization, using 0.25 % trypsin in PBS containing 0.02 % EDTA (Biochrom KG L 2163). For each experiment, approximately 10.000 cells were seeded with 0.1 mL culture medium per well of the 96-well microplates (NUNC 167008).

*Cytotoxic assay.* For the cytotoxic assay, HeLa cells were pre-incubated for 48 hours without the test substances. The dilutions of the compounds were carried out carefully on the subconfluent monolayers of HeLa cells after the pre-incubation time. Cells were incubated with dilutions of the test substances for 72 hours at 37 °C in a humidified atmosphere and 5 % CO₂.

*Method of evaluation.* For estimating the influence of chemical compounds on cell proliferation of K-562, the numbers of viable cells present in multiwell plates were determined via CellTiter-Blue^{®} assay. The indicator dye resazurin was used to measure the metabolic capacity of cells as an indicator of cell viability. Viable cells of untreated control retain the ability to reduce resazurin into resorufin, which is highly fluorescent. Nonviable cells rapidly lose metabolic capacity, do not reduce the indicator dye, and thus do not generate a fluorescent signal. Under our experimental conditions, the signal from the CellTiter-Blue® reagent is proportional to the number of viable cells. The adherent Huvec and HeLa cells were fixed by glutaraldehyde and stained with a 0.05 % solution of methylene blue for 15 min. After gentle washing the stain was eluted with 0.2 mL of 0.33 N HCl in the wells. The optical densities were measured at 660 nm in SUNRISE microplate reader (TECAN). The Gl₅₀ and CC₅₀ values were defined as being where the dose response curve intersected the 50% line, compared to untreated control. The comparisons of the different values were performed with software Magellan (TECAN).

### References:

1. Strobel, G.; Daisy, B.; Castillo, U.; Harper, J., Natural products from endophytic microorganisms. J. Nat. Prod. 2004, 67, (2), 257-268.
2. Ryan, R. P.; Germaine, K.; Franks, A.; Ryan, D. J.; Dowling, D. N., Bacterial endophytes: recent developments and applications. FEMS Microbiol. Lett. 2008, 278, (1), 1-9.
3. Strobel, G.; Daisy, B., Bioprospecting for microbial endophytes and their natural products. Microbiol. Mol. Biol. Rev. 2003, 67, (4), 491-502.
4. Strobel, G., Harnessing endophytes for industrial microbiology. Curr. Opin. Microbiol. 2006, 9, (3), 240-244.
5. Zhang, H. W.; Song, Y. C.; Tan, R. X., Biology and chemistry of endophytes. Nat. Prod. Rep. 2006, 23, (5), 753-771.
6. Kogel, K. H.; Franken, P.; Huckelhoven, R., Endophyte or parasite--what decides? Curr. Opin. Plant Biol. 2006, 9, (4), 358-363.
7. Horiuchi, M.; Seyama, Y., Antiinflammatory and Antiallergic Activity of Bidens pilosa L. var. radiata SCHERFF. J. Health Sci. 2006, 52, (6), 711-717.
8. Oliveira, F. Q.; Andrade-Neto, V.; Krettli, A. U.; Brandao, M. G., New evidences of antimalarial activity of Bidens pilosa roots extract correlated with polyacetylene and flavonoids. J. Ethnopharmacol. 2004, 93, (1), 39-42.
9. Deba, F.; Xuan, T. D.; Yasuda, M.; Tawata, S., Herbicidal and fungicidal activities and iidentification of potential phytotoxins from Bidens philosa L. var. radiata Scherff. Weed Biol. Management 2007, 7, 77-83.
10. Schett, G.; Casati, B.; Willinger, B.; Weinländer, G.; Binder, T.; Grabenwöger, F.; Sperr, W.; Geissler, K.; Jäger, U., Endocarditis and Aortal Embolization Caused by Aspergillus terreus in a Patient with Acute Lymphoblastic Leukemia in Remission: Diagnosis by Peripheral-Blood Culture. J. Clin. Microbiol. 1998, 36, (11), 3347-3351.
11. van Burik, J. H.; Colven, R.; Spachi, D. H., Cutaneous Aspergillosis. J. Clin. Microbiol. 1998, 36, (11), 3115-3121.
12. Vennewald, I.; Wollina, U., Cutaneous infections due to opportunistic molds: uncommon presentations. Clin. Dermatol. 2005, 23, 565-571.
13. Cooke, F.J.; Terpos, E.; Boyle, J.; Rahemtulla, A.; Rogers, T.R., Dissiminated Aspergillus terreus infection arising from cutaneous inoculation treated with caspofungin. Clin Microbiol. Infect. 2003, 9, 1238-1241.
14. Saldanha, R. L.; Garcia, J. E.; Dekker, R. F. H.; Vilas-Boas, L. A.; Barbosa, A. M., Genetic diversity among Botryosphaeria isolates and their correlation with cell wall-lytic enzyme production. Braz. J. Microbiol. 2007, 38, 259-264.
15. Jacobs, K. A.; Rehner, S. A., Comparison of cultural and morphological characters and ITS sequences in anamorphs of Botryosphaeria and related taxa. Mycologia 1998, 90, (4).
16. Müller, K., Pharmaceutically relevant metabolites from lichens. Appl. Microbiol. Biotechnol. 2001, 56, (1-2), 9-16.
17. Neamati, N.; Hong, H.; Mazumder, A.; Wang, S.; Sunder, S.; Nicklaus, M. C.; Milne, G. W.; Proksa, B.; Pommier, Y., Depsides and depsidones as inhibitors of HIV-1 integrase: discovery of novel inhibitors through 3D database searching. J. Med. Chem. 1997, 40, (6), 942-951.
18. Fernandez, E.; Reyes, A.; Hidalgo, M. E.; Quilhot, W., Photoprotector capacity of lichen metabolites assessed through the inhibition of the 8-methoxypsoralen photobinding to protein. J. Photochem. Photobiol. B 1998, 42, (3), 195-201.
19. Rosenblueth, M.; Martinez-Romero, E., Bacterial endophytes and their interactions with hosts. Mol. Plant Microbe Interact 2006, 19, (8), 827-837.

## Claims

1. A compound of formula (I): 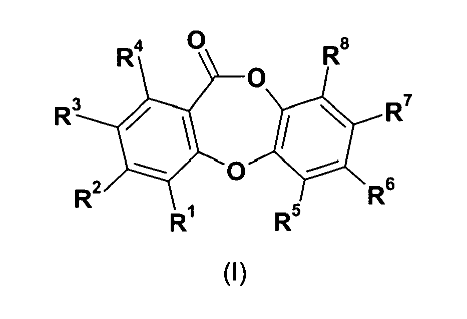 wherein
R¹ is a group of formula CHO or CH₂OH;
R² is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R³ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁴ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁵ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁶ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁷ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, and
R⁸ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, or
or wherein two of the groups R², R³, R⁴, R⁵, R⁶ R⁷ and R⁸ together are part of a cycloalkyl, a heterocycloalkyl, an alkylcycloalkyl, a heteroalkylcycloalkyl, an aryl, a heteroaryl, an aralkyl or a heteroaralkyl group;
with the provision that at least one of R⁵ or R⁸ is a hydrogen atom when R¹ is CHO,
or a pharmacologically acceptable salt, solvate, hydrate or a pharmacologically acceptable formulation thereof.

2. Compound according to claim 1, wherein R¹ is an aldehyde (CHO) group.

3. Compound according to claim 1 or 2, wherein R² is a hydroxy or a methoxy group, especially a hydroxy group.

4. Compound according to anyone of the preceding claims, wherein R³ is a hydrogen atom or a halogen atom (e.g. F or Cl), more preferably, H or Cl, especially a hydrogen atom.

5. Compound according to anyone of the preceding claims, wherein R⁴ is a C₁-C₄ alkyl group or a C₂-C₄ alkenyl group, especially a methyl group.

6. Compound according to anyone of the preceding claims, wherein R⁵ is a C₁-C₄ alkyl group, especially a methyl group and R⁸ is a hydrogen atom.

7. Compound according to anyone of the preceding claims, wherein R⁶ is a hydrogen atom, a halogen atom (e.g. F or Cl), CHO or COOH, especially hydrogen.

8. Compound according to anyone of the preceding claims, wherein R⁷ is a hydroxy or a methoxy group, especially a hydroxy group.

9. Compound of formula (Ia) 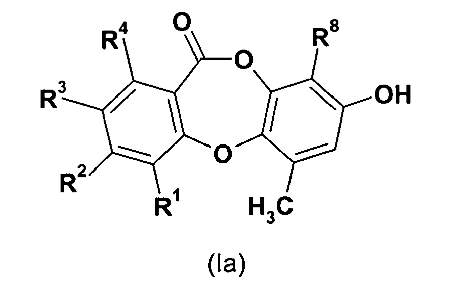 wherein
R¹ is a group of formula CHO or CH₂OH;
R² is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R³ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁴ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R⁸ is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
or wherein two of the groups R², R³, R⁴ and R⁸ together are part of a cycloalkyl, a heterocycloalkyl, an alkylcycloalkyl, a heteroalkylcycloalkyl, an aryl, a heteroaryl, an aralkyl or a heteroaralkyl group,
or a pharmacologically acceptable salt, solvate, hydrate or a pharmacologically acceptable formulation thereof for use as a medicament.

10. A compound according to claim 10, wherein R² is a hydroxy or a methoxy group, especially a hydroxy group.

11. Compound according to claim 9 or 10, wherein R³ is a hydrogen atom or a halogen atom (e.g. F or Cl), more preferably, H or Cl, especially a hydrogen atom.

12. Compound according to claim 9, 10 or 11, wherein R⁴ is a C₁-C₄ alkyl group or a C₂-C₄ alkenyl group, especially a methyl group.

13. Compound according to anyone of claims 9 to 12, wherein R⁸ is a hydrogen atom or a C₁-C₄ alkyl group, especially hydrogen or a methyl group.

14. Pharmaceutical composition comprising a compound according to anyone of the preceding claims and optionally one or more carrier substances and/or one or more adjuvants.

15. Compound or pharmaceutical composition according to anyone of the preceding claims for the treatment of bacterial infections, fungal diseases or cancer.
